# EUROPÄISCHE PATENTSCHRIFT

(11) **EP 2 593 058 B1**
(45) Veröffentlichungstag und Bekanntmachung des Hinweises auf die Patenterteilung: **07.03.2018**
(21) Anmeldenummer: 11741541.4
(22) Anmeldetag: 11.07.2011
(51) Int. Cl.: A61F 13/02, A61M 27/00

(54) **WUNDPFLEGEARTIKEL ZUR WUNDBEHANDLUNG UNTER EINSATZ VON UNTERDRUCK ENTHALTEND MIKROFASERN**
WOUND CARE ITEM FOR TREATING A WOUND USING NEGATIVE PRESSURE AND CONTAINING MICROFIBERS
ARTICLE POUR LE TRAITEMENT DE PLAIES PAR L'UTILISATION DE MICROFIBRES CONTENANT UNE PRESSION NÉGATIVE

(30) Priorität: 13.07.2010 DE 102010036371
(43) Veröffentlichungstag der Anmeldung: 22.05.2013
(73) Patentinhaber: BSN medical GmbH, 20253 Hamburg (DE)
(72) Erfinder: RIESINGER, Birgit, 48149 Münster (DE)
(74) Vertreter: Jostarndt Patentanwalts-AG
(86) Internationale Anmeldenummer: PCT/EP2011/061755
(87) Internationale Veröffentlichungsnummer: WO 2012/007424

(56) Entgegenhaltungen:
- WO-A1-2006/048246
- WO-A1-2009/071938
- WO-A2-2009/012438
- DE-U1-202006 012 210
- US-A1- 2008 177 253
- US-A1- 2010 036 334

## Beschreibung

Die Erfindung betrifft einen Wundpflegeartikel gemäß dem Oberbegriff des Anspruchs 1.

Bei der Unterdruck Therapie zur Wundheilung soll durch Ausübung eines Unterdruckes auf die Wunde der Wundverschluss erleichtert bzw. beschleunigt werden, wodurch die Behandlungszeit verkürzt werden kann. Mittels einer Pumpe wird ein kontrollierter, örtlich begrenzter negativer Druck in einer Wunde erzeugt und dadurch der Heilungsprozess in chronischen Wunden beschleunigt. Zudem wird Wundsekret abgesaugt, womit eine Säuberung der Wunde einhergeht, ein bestehendes Wundödem wird verkleinert und die Durchblutung in der Wunde wird durch den Unterdruck gefördert.

Herkömmliche Wundpflegeartikel zur Unterdruck Therapie sind beispielsweise aus der US 5636643 und der US7216651 bekannt. Diese bestehen aus einem offenzelligen Polymer Schaumstoff, der auf die Wunde aufgelegt wird, einer darüber befestigten Folie, die auf die umgebenden Haut aufgeklebt wird, sowie einem Gerät, das den Unterdruck erzeugt.

Nachteilig an derartigen Wundpflegeartikel zur Unterdruck Therapie ist jedoch, dass sich der verwendete Schaumstoff durch das Anlegen des Unterdrucks erheblich versteift und somit für den Patienten höchst unangenehm ist. Im schlimmsten Fall kann ein Verrutschen des versteiften Schaumstoffs sogar zu einem weiteren Aufscheuern der Wunde führen.

Zudem ist es notwendig den Schaumstoff vor dem Anlegen des Wundpflegeartikels entsprechend der Form der jeweiligen Wunde zuzuschneiden. Folglich ist die Verwendung der bekannten Wundpflegeartikel zur Unterdruck Therapie relativ zeitaufwendig.

### ZUSAMMENFASSUNG DER ERFINDUNG

Aufgabe der vorliegenden Erfindung ist es daher, einen Wundpflegeartikel bereit zu stellen, der die genannten Nachteile der Produkte aus dem Stand der Technik nicht aufweist.

Insbesondere ist es Aufgabe der vorliegenden Erfindung einen Wundpflegeartikel zur Unterdruck Therapie mit einem erhöhten Tragekomfort für den Patienten bereitzustellen.

Zudem ist es Aufgabe der vorliegenden Erfindung einen Wundpflegeartikel zur Unterdruck Therapie bereitzustellen, der mit geringerem Zeitaufwand verwendbar ist.

Diese Aufgaben werden mit einem Wundpflegeartikel gemäß dem vorliegenden Hauptanspruch gelöst; die Unteransprüche geben bevorzugte Ausführungsformen an. Dabei sind durch Zahlenwerte begrenzte Wertebereiche stets einschließlich der genannten Zahlenwerte zu verstehen.

### KURZE BESCHREIBUNG DER ZEICHNUNGEN

Fig. 1 zeigt einen nicht zur Erfindung gehörenden Wundpflegeartikel 100 vor der Erzeugung eines Unterdrucks.
Fig. 2 zeigt einen nicht zur Erfindung gehörenden Wundpflegeartikel 200 nach der Erzeugung eines Unterdrucks.
Fig. 3 zeigt einen nicht zur Erfindung gehörenden Wundpflegeartikel 300 umfassend einen Verbindungsschlauch 305 für eine Vakuumpumpe vor der Erzeugung eines Unterdrucks.
Fig. 4 zeigt einen erfindungsgemäßen Wundpflegeartikel 400 umfassend einen Absorptionskörper 405 aufweisend superabsorbierende Polymere vor der Erzeugung eines Unterdrucks.
Fig. 5 zeigt einen nicht zur Erfindung gehörenden Wundpflegeartikel 500 der wurstförmig ausgestaltet ist und ein dreidimensionales Wunddistanzgitter 502 umfasst.

### AUSFÜHRLICHE BESCHREIBUNG DER ERFINDUNG

Erfindungsgemäß wird ein Wundpflegeartikel zur Wundbehandlung unter Einsatz von Unterdruck bereitgestellt, aufweisend:
- ein Wirkelement aufweisend Mikrofasern, und
- eine Einrichtung, über die in der Wunde befindliche Stoffe abgesaugt werden können.

Der Begriff "Wundpflegeartikel" bezeichnet, neben klassischen primären und sekundären Wundauflagen, auch Wundpflegetücher, Tupfer, Tampons, Wundtamponaden und dergleichen. Diese können absorbierende und nicht absorbierende Eigenschaften haben.

Unter dem Begriff "Wirkelement" soll im Folgenden eine Einrichtung verstanden werden, die die Wunde gegenüber der Wundabdeckung und der Einrichtung, über die unter der Wundabdeckung befindliche Stoffe abgesaugt werden können, abschirmt, so dass die Wunde nicht mit der Wundabdeckung verkleben kann und es zu keiner Beeinträchtigung der Wunde durch ein Absaugen von Stoffen kommen kann.

Unter dem Begriff "Mikrofaser" soll im Folgenden eine Sammelbezeichnung für Fasern verstanden werden, deren Einzelfäden feiner als 1 dtex sind, also ein Gewicht von maximal 1 Gramm pro 10.000 Meter aufweisen.

Aufgrund dieser Größendefinition sind Naturfasern wie Cellulose oder Baumwolle, die normalerweise in Wundpflegeartikeln Verwendung finden, durch den Begriff "Mikrofasern" nicht erfasst. So weist ein Baumwollfaden mindestens 2 dtex auf.

Es ist jedoch denkbar, Fasern aus Naturstoffen zu denen neben Cellulose und Baumwolle z.B. auch Flachs, Leinen, Wolle und Seide gehören soweit zu modifizieren, dass diese aufgrund ihrer Größe unter den Begriff "Mikrofasern" fallen.

Insbesondere fallen auch sogenannte "Nanofasern" unter die Definition des Begriffs "Mikrofasern". Nanofasern können insbesondere mit sogenannten Elektrospinnverfahren hergestellt werden, wie sie z.B. in Greiner und Wendorff (2007), Angewandte Chemie 119 (30), 5770 beschrieben sind. Dabei können die Fasern aus synthetischen Polymeren oder Harzen bestehen, jedoch auch aus natürlichen Polymeren, beispielsweise auf Poly(L)-Lactid-Basis. Ebenso kann es sich bei besagten Nanofasern um Seiden-Nanofasern handeln. Hierbei werden kugelförmige Seiden-Nanopartikel mit einem Durchmesser von 35-125 nm erzeugt. Die feinen kristallinen Seidenproteinkugeln werden durch die Verwendung von mit Wasser mischbaren Lösungsmitteln direkt aus der umgewandelten Flüssigseide hergestellt.

Unter dem Begriff "Einrichtung über die in der Wunde befindliche Stoffe abgesaugt werden können" soll im Folgenden eine Einrichtung mit Saugkraft verstanden werden.

Unter dem Begriff "Stoffe" sollen im Folgenden Gase und/oder Wundsekrete verstanden werden.

Überraschender Weise wurde nun festgestellt, dass ein Wirkelement, welches Mikrofasern aufweisen, auch bei angelegtem Unterdruck nicht steif wird und somit den Tragekomfort des Wundpflegeartikels deutlich erhöht.

In einer bevorzugten Ausführungsform umfassend der erfindungsgemäße Wundpflegeartikel eine Wundabdeckung, mit der eine Wunde eines Patienten gasdicht verschließbar ist, dadurch gekennzeichnet, dass
- das Wirkelement unterhalb der Wundabdeckung anordbar ist,
- die Einrichtung in der Wundabdeckung umfasst ist und so unter der Wundabdeckung befindliche Stoffe abgesaugt werden können.

Bei der Behandlung der meisten Wunden unter Einsatz von Unterdruck ist es vorteilhaft, wenn eine Wundabdeckung den Wundraum gasdicht gegenüber der Umgebung verschließt, da so der Unterdruck am ehesten konstant aufrecht erhalten werden kann.

Unter dem Begriff "Wundabdeckung" soll im Folgenden eine Einrichtung des erfindungsgemäßen Wundpflegeartikel verstanden werden, die die zu behandelte Wunde gasdicht gegen die Umwelt verschließt. Daher musst die Wundabdeckung auf bzw. um die Wunde herum angeordnet sein und ein gasdichtes Material umfassen. Die Wundabdeckung kann dehnbar ausgestaltet sein.

Vorzugsweise besteht die Wundabdeckung aus folienartigem Material, das derart steif ist, dass unter normalen Bedingungen, d. h. im nicht benutzten und im am Körper des Patienten angelegten Zustand nicht schrumpft.

Ferner ist bevorzugt, dass die Wundabdeckung einen Kleberand aufweist, mit dem sie auf der Haut, die die Wunde umgibt, befestigt werden kann.

Unter dem Begriff "Einrichtung, die in der Wundabdeckung umfasst ist" soll im Folgenden eine bevorzugte Ausfiihrungsform der Einrichtung über die in der Wunde befindliche Stoffe abgesaugt werden können verstanden werden, also eine Einrichtung über die **unter** der Wundabdeckung befindliche Stoffe abgesaugt werden. Beispielsweise kann es sich hierbei um eine Einstichstelle für eine Nadel oder einen Schlauchanschluss handelt. Ferner kann ein Schlauch auch bereits fest mit der Wundabdeckung verbunden sein. Überdies ist es auch denkbar, dass an der Einrichtung, über die in der Wunde befindliche Stoffe abgesaugt werden können, mehr als ein Schlauch angeschlossen ist.

Ferner zeichnen sich Mikrofasern durch
a) eine große Weichheit und Bauschigkeit,
b) eine Abrasivität gegenüber mikrobiellen Biofilmen, die durch ihre außerordentliche Feinheit und Oberflächenhärte bedingt ist,
c) eine gute Wärmeisolation, und
d) eine geringe Haftungsneigung an der Wundoberfläche aus.

Besonders bevorzugt werden synthetische und somit hydrophobe Mikrofasern so z.B. Fasern aus Polyolefinen wie Polyester, Polyamid, oder Fasern aus Acryl, denn diese zeigen eine geringe Feuchtigkeitsaufnahme bei gleichzeitiger Fähigkeit, Feuchtigkeit abzuleiten. Daher bleiben keine Wundsekrete an diesen Fasern hängen, was die Ausbildung eines das Bakterienwachstum fördernden Mikroklimas innerhalb des Wundpflegeartikels verhindert.

Weiterhin wird durch die geringe Feuchtigkeitsaufnahme eine laterale Diffusion von Feuchtigkeit innerhalb des Wundpflegeartikels verhindert, was ansonsten zu einer Mazeration der die Wunde umgebenden gesunden Haut fuhren könnte, falls mit den Wundrändern in Kontakt stehende Abschnitte feucht sind.

Trotz langjähriger Bekanntheit von Mikrofasern und deren Eignung für Haushalts- und Hygienezwecke ist bislang nicht vorgeschlagen worden, Mikrofasern umfassend einen Wundsekrete absorbierenden Absorptionskörper auch als Wirkelement für die Wundversorgung und speziell für die Wundbehandlung unter Einsatz von Unterdruck zu verwenden.

Die große Weichheit und Bauschigkeit macht Mikrofasern insbesondere geeignet für polsternde Zwecke, wie sie in der zur Wundbehandlung unter Einsatz von Unterdruck vonnöten sind, da Wunden in der Regel stark berührungsempfindlich sind und dennoch ein gewisser Druck ausgeübt werden muss um ein Überwachsen des Wundgewebes zu verhindern. Die Bauschigkeit der Fasern geht auf Formgedächtniseigenschaften zurück, die dafür sorgen, dass die Fasern nach Kompression stets in ihre ursprüngliche Form zurückkehren und so ihre polsternden Eigenschaften bewahren. Die große Weichheit geht insbesondere auf die außerordentliche Feinheit der Fasern zurück, die von der menschlichen Haut kaum oder als sehr weich wahrgenommen werden.

Besagte Anordnung der Wundabdeckung auf bzw. um die Wunde herum kann auf verschiedene Arten erfolgen. So kann vorgesehen sein, dass die Wundabdeckung direkt mit der die Wunde umgebenden Haut verklebt wird, oder das zusätzliche Mittel zur Befestigung verwendet werden.

Bevorzugt umfasst das Wirkelement aufweisend Mikrofasern auch eine Stützeinrichtung, die einem Steifwerden des Wirkelements zusätzlich entgegenwirkt. Diese Stützeinrichtung ist vorzugsweise als Stützskelett ausgestaltet.

In einer bevorzugten Ausführungsform umfasst der Wundpflegeartikel ferner wenigstens ein Unterdruck erzeugendes Mittel.

Unter dem Begriff "Unterdruck erzeugendes Mittel" soll im Folgenden eine Einrichtung verstanden werden, die den Unterdruck erzeugt. In der einfachsten Ausführungsform handelt es sich hierbei um eine Injektionsspritze oder eine Redonflasche. Mit dieser wird, wenn der Wundpflegeartikel am Patienten angelegt ist, die Wundabdeckung an der Einrichtung, über die unter der Wundabdeckung befindliche Stoffe abgesaugt werden können, durchstochen und Gase von unterhalb der Wundabdeckung abgesaugt.
Bevorzugt ist jedoch vorgesehen, dass es sich bei dem Unterdruck erzeugenden Mittel um eine Vakuumpumpe z.B. eine elektrische oder eine handbetriebene Vakuumpumpe handelt.

Es ist selbstverständlich denkbar, dass der erfindungsgemäße Wundpflegeartikel mehr als ein Unterdruck erzeugendes Mittel aufweist.

In einer weiteren bevorzugten Ausführungsform steht das Wirkelement aufweisend Mikrofasern mindestens auf einer Seite mindestens abschnittsweise in Kontakt mit einem Wunddistanzgitter.

Der Begriff "Wunddistanzgitter", bezeichnet ein gitter- oder gazeartiges Gebilde, dass - häufig als sogenannter "Primärverband" - unmittelbar auf eine Wunde aufgelegt wird, bevor ggf. ein Sekundärverband aufgelegt wird. Wunddistanzgitter werden häufig auch als "Wundgazen" bezeichnet und dienen primär dazu, ein Verkleben der gesamten Wundauflage mit der Wunde zu verhindern. Hierfür sind Wunddistanzgitter aus einem Material gefertigt oder mit einem solchen beschichtet, dass eine Haftung an der Wunde reduziert bzw. verhindert. Alternativ kann auch die Geometrie des Wunddistanzgitters so ausgestaltet sein, dass eine Haftung an der Wunde reduziert bzw. verhindert wird.

Handelsübliche Beispiele für Wunddistanzgitter sind z.B. die Produkte "Mepitel" (silikonbeschichtetes, elastisches Polyamidnetz), "Physiotulle", "Urgotül" (Netz aus Polyesterfasern getränkt mit Hydrokolloidpartikeln und Vaseline) und "ADAPTIC" (glattes Viskosegewirk, imprägniert mit einer Öl-in-Wasser-Emulsion).

Bei den vorgenannten Produkten handelt es sich um zweidimensionale Wunddistanzgitter, die also im Wesentlichen die Form einer Gaze oder eines Netzes annehmen. Ein dreidimensionales Wunddistanzgitter ist unter dem Namen "sorbion plus" bekannt und insbesondere in der EP2004116 der Anmelderin der vorliegenden Erfindung beschrieben, auf deren Inhalt hier vollumfänglich verwiesen wird.

Ein solches dreidimensionales Wunddistanzgitter weist einen flüssigkeitsdurchlässigen, schmiegsamen Materialabschnitt aus einem Thermoplasten auf, der eine erste, glatte Oberfläche, eine der glatten Oberfläche abgewandte und eine zweite raue Oberfläche aufweist. Letztere wird erzeugt durch eine Vielzahl von dreidimensionalen Perforationen, deren Wände von der ersten, glatten Oberfläche beginnend auskragen und jeweils in einen Randüberstand mit freier Kante auslaufen, womit sie der zweiten Oberfläche einen rauen Griff verleihen.

Die Perforationen werden durch einseitig eingefügte Starrzungen hergestellt. Es entsteht so eine rauhe Seite mit Auskragungen sowie eine mehr oder minder glatte Seite.

Ein Wunddistanzgitter verhindert zuverlässig ein Verkleben des Wundpflegeartikels mit der Wunde und Wundrandmazeration. Daher muss, anders als bei den aus dem Stand der Technik bekannten Schäumen das besagte Wirkelement nicht mehr auf die Form der Wunde zugeschnitten werden, was den Arbeitsaufwand des medizinischen Personals erheblich reduziert.
In einer bevorzugten Ausfiihrungsform umgibt das Wunddistanzgitter das Wirkelement aufweisend Mikrofasern in Form einer Hülle oder stellt einen Teil einer solchen Hülle dar.

In einer derartigen Ausführungsform spielt es mithin keine Rolle mit welcher Seite das Wirkelement auf die Wunde aufgelegt wird.

In einer alternativen bevorzugten Ausführungsform ist das Wunddistanzgitter flächig zwischen Wunde und dem Wirkelement aufweisend Mikrofasern angeordnet.

Demgemäß ist es auch möglich, dass das Wunddistanzgitter separat vorliegt und als erstes - vor dem Wirkelement - auf die Wunde aufgelegt wird.

Vorzugsweise liegt das Wirkelement aufweisend Mikrofasern in mindestens einer Form vor ausgewählt aus der Gruppe enthaltend
- Mikrofaserwatte, ggf. umgeben von einer Hülle
- Mikrofaservlies
- Mikrofasertuch, und/oder
- Mikrofaserschaum.

Der Begriff "Vlies" bezeichnet ein textiles Flächengebilde aus einzelnen Fasern, das im Gegensatz zu Geweben, Gestricken und Gewirken nicht aus Garnen hergestellt wird. Vliese behalten ihre strukturelle Integrität i.d.R. durch Haftung der einzelnen Fasern aneinander. Sie werden auch als "Nonwovens" bezeichnet, und z.B. durch Walken der Fasern hergestellt.

Mikrofaser-Gewebe können geraut oder nicht geraut sein und werden z. T. auf feinste Trägergewebe kaschiert. Mikrofaser-Gewirke können ebenfalls gerauht oder nicht gerauht sein und werden z.T. als sehr feine Kettengewirke auf einem Trägergewebe stabilisiert

In einer bevorzugten Ausführungsform liegt das Wirkelement aufweisend Mikrofasern wurst-, kissen-, quader-, prisma- oder tuchförmig vor.

Die Ausgestaltung des Wirkelements hängt unter anderem von den Gegebenheiten der jeweiligen Wunde ab. Wenn es beispielsweise vorteilhaft ist auf eine bestimmte Wunde möglichst viel Druck auszuüben z.B. um Wundrandmazeration zu verhindern, würde sich eine wurstförmige Ausgestaltung anbieten. Die genaue Zusammensetzung des Wundpflegeartikels spielt bei der Auswahl der Wirkelementausführung jedoch ebenso eine Rolle.

Gemäß der Erfindung umfasst das Wirkelement aufweisend Mikrofasern auch ein Wundsekrete absorbierendes Absorptionsmaterial und der Wundpflegeartikel weist einen Wundsekrete absorbierenden Absorptionskörper auf.

Das Absorptionsmaterial bzw. der separate Absorptionskörper bewirken, dass Wundsekrete nicht nur abgesaugt werden können, wie es in bestimmten oben beschriebenen Ausführungsformen der Fall ist, sondern auch absorbiert werden können.

Diese Absorptionsfähigkeit ist besonders wichtig, falls das Unterdruck erzeugende Mittel keine Wundsekrete sondern nur Gase absaugt, da nur durch Absorption der Wundsekrete eine gute Wundheilung gewährleistet werden kann.

Zudem können das Absorptionsmaterial bzw. der separate Absorptionskörper als Rücklaufschutz dienen, falls das Unterdruck erzeugende Mittel zeitweise nicht funktioniert, z.B. wenn eine Vakuumpumpe abgeschaltet wird oder ausfällt beispielsweise aufgrund eines Flaschenwechsels, eines Defekts oder eines Stromausfalls. In einem solchen Fall ist es äußerst wichtig, dass die abgesaugten Wundsekrete (Wundexsudat) nicht in die Wunde zurückfließt, da sie bereits kontaminiert sind und somit zu erheblichen Komplikationen in der Wundheilung führen könnten, falls sie in die Wunde zurück gelangen.

Der Begriff "Exsudat" bezeichnet eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nichtentzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert sind und sowohl bestehendes als auch neu gebildetes Collagen in der Wunde abbauen.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger als drei Tage andauernde exsudative Phasen als Komplikation und spricht von einer pathologischen Exsudation, welche zu einer Chronifizierung der Wunde beitragen kann. Die zugrunde liegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln.

Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert. Ein besonders gravierender Unterschied ergibt sich hinsichtlich der Aktivität der zuvor angesprochenen Matrix-Metalloproteasen. Neben der Vorbereitung des Wundbetts sind sie auch beim späteren Umbau des Granulations- zum Narbengewebe beteiligt. Diese Enzyme werden normalerweise als inaktives Präenzym gebildet und in ihrer Aktivierung durch entsprechende Inhibitoren reguliert (tissue inhibitors of metalloproteases, TIMPs), welche gleichzeitig selbst eine positive Wirkung auf das Zellwachstum haben. Im chronischen Exsudat scheint aufgrund von Störungen in diesem Regulationssystem die Aktivität der Proteasen erhöht, was möglicherweise zu einer Regression der Wundheilung beiträgt. Das pathologische Exsudat ist hinsichtlich des Gehalts seiner Komponenten aus dem der wundprogression förderlichen Gleichgewicht geraten. Daraus ergeben sich verschiedene Komplikationen, die zur weiteren Verschlechterung und Chronifizierung der Wunde beitragen. Folglich ergibt sich die Notwendigkeit dieses Exsudat aus der Wunde zu entfernen.

Außerdem kann unter Verwendung von Absorptionsmaterial bzw. einem separaten Absorptionskörper ein Unterdruck nur zu Beginn der Behandlung angelegt werden und im weiteren Verhandlungsverlauf das Unterdruck erzeugende Mittel abgeschaltet werden. Nach Abschaltung hält das Absorptionsmaterial bzw. der separate Absorptionskörper die Saugkraft aufrecht, die für die Unterdruck Therapie erforderlich ist. Diese Vorgehensweise ist bei Wunden, die zu Nekrosen neigen, vorteilhaft, da sie eine schonendere Unterdrucktherapie ermöglicht.

Falls ein separater Absorptionskörper verwendet wird, ist das Wirkelement vorzugsweise kissenförmig ausgestaltet, da der Absorptionskörper, besonders nachdem er angefangen hat Wundsekrete zu absorbieren, bereits Druck auf die Wunde ausübt und dies nicht mehr durch die Ausgestaltung des Wirkelements geschehen muss.

Da das Wirkelement selbst das Wundsekrete absorbierendes Absorptionsmaterial umfasst, sind die oben bereits erwähnten feuchtigkeitsleitenden Eigenschaften der Mikrofasern besonders von Vorteil. In einem solchen Fall sind die Mikrofaser bevorzugt auf der der Wunde zugewandten Seite des Wirkelements angeordnet. Auf diese Weise passiert austretendes Exsudat den Mikrofaserabschnitt (der wie eine Art Transitschicht fungiert, ähnlich wie dies von Sportunterwäsche bekannt ist) schnell und wird in den absorbierenden Abschnitt transportiert; der in Kontakt mit der Wunde stehende Abschnitt enthaltend Mikrofasern bleibt trocken.

Hinzu kommt, dass ein Wundpflegeartikel aufgrund der geringeren Feuchtigkeitsaufnahme in den Abschnitten enthaltend Mikrofasern dort im Gegensatz zu Wundpflegeartikeln mit z.B. feuchtigkeitsabsorbierenden Zellulosefasern bei Kontakt mit Flüssigkeit nicht oder weniger stark kollabiert, und so die strukturelle Integrität des Wundpflegeartikels gewahrt bleibt, was insbesondere die polsternden Eigenschaften aufrecht erhält.

Zudem eignet sich ein Wirkelement das außer Mikrofasem auch Absorptionsmaterial aufweist als Wundfüller. Oftmals gehen chronische Wunden mit einem erheblichen temporären Gewebsverlust einher, der ausgefüllt werden sollte, um einerseits die Weiterleitung des am Wundboden austretenden Wundexsudats beispielsweise an ein darüber liegendes absorbierendes Absorptionsmaterial zu ermöglichen (Dochtwirkung), und andererseits die Wunde, die extrem druck- und schmerzempfindlich ist, sogar unter Unterdruckbedingungen weich auszupolstern. Die Funktion als Wundfüller ist insbesondere aus psychologischen Gründen wichtig, da besagter Gewebsverlust für den Patienten eine erhebliche Belastung darstellt. Ein solcher Wundfüller kann daher auch als Kurzzeitprothese verstanden werden, die dem Patienten das Gefühl vermittelt, der Gewebsverlust sei - zumindest kurzfristig - behoben. Das erfindungsgemäß vorgeschlagene Wirkelement eignet sich aus den genannten Gründen - insbesondere da es unter Unterdruck Bedingungen nicht steif wird, sondern elastisch also weich und polsternd bleibt - hervorragend zu diesem Zweck.

Bevorzugt ist vorgesehen, dass das Absorptionsmaterial und/oder der Absorptionskörper hydroaktive Polymere aufweisen.

Unter dem Begriff "hydroaktive Polymere" sollen im folgenden Polymere verstanden werden, die feuchtigkeitsbindende Eigenschaften haben. Hierzu zählen u. a.
- Agar,
- Carrageen,
- Johannisbrotkernmehl,
- Guarkernmehl,
- Traganth,
- Gummi arabicum, d
- Xanthan,
- Karaya,
- Tarakernmehl,
- Gellan,
- Pektin,
- Chitosan,
- Hyaluronsäure,
- Modifizierte Stärke, sowie
- Zellulose und Zelluloseether, wie z. B.
- Carboxymethylzellulose,
- Natriumcarboxymethylzellulose
- Hydroxypropylzellulose,
- Hydroxypropylmethylzellulose,
- Methylzellulose,
- Methylethylzellulose,
- Offenzellige Schäume aus Polyurethan und
- Alginate.

Die genannten hydroaktiven Polymere nehmen wässrige Flüssigkeiten auf und bilden dabei eine feuchte Oberfläche aus. Ggf. nehmen sie eine gelartige Form an. Die feuchte Oberfläche bzw. die Gelform trägt dazu bei, die Wundhaftneigung des Absorptionskörpers zu reduzieren, so dass dieser sich nach Verwendung ggf. atraumatisch und schmerzlos ablösen lässt. Durch die Gelform weist der Wundpflegartikel eine kühlende und somit schmerzlindernde Wirkung auf. Überdies ermöglicht die Gelform ggf. die Ausbildung eines Wundheilungsfördernden Feuchtklimas.

Die Verwendung von hydroaktiven Polymeren wird bevorzugt, da sie die absorbierenden Eigenschaften des Absorptionsmaterial und/oder des Absorptionskörper besonders gut unterstützen.

In einer besonders bevorzugten Ausführungsform handelt es sich bei den hydroaktiven Polymeren um superabsorbierende Polymere, da diese die Saugkraft des Unterdruck erzeugenden Mittels besonders gut unterstützen bzw. diese aufrecht erhalten können.

Unter dem Begriff "superabsorbierende Polymere" werden im Folgenden Kunststoffe verstanden, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Flüssigkeiten in den Polymerpartikel quillt er auf und strafft auf molekularer Ebene dieses Netzwerk, so dass das Wasser ohne Hilfe nicht mehr entweichen kann.

Die Superabsorbierenden Polymere (SAP) können in Form einer Schüttung vorliegen, wobei der Begriff "Schüttung" sowohl Granulate und Pulver als auch Materialstücke, wie Schaumstücke, umfassen kann.
Die Superabsorber-Teilchen können in Pulver- oder Granulatform in einer Partikelgröße zwischen 100 und etwa 1000 µm vorliegen.

Ebenso ist jedoch bei Superabsorbierenden Polymeren die Faserform besonders bevorzugt, da es sich hierbei um ein sowohl im trockenen als auch im gequollenen Zustand sehr weiches Produkt handelt, das modellierbar und nicht steif ist und das im Gegensatz zu den in Granulat- oder Pulverform vorliegenden superabsorbierenden Polymeren überdies eine geringe Abrasivität aufweist. Dies gilt sowohl für Fasern als solches als auch für Fasergewirke, -gelege oder -vliese und/oder Faserwatte.

Die vorgeschlagene Verwendung von Superabsorbierenden Polymeren in Faser- oder Garnform weist gegenüber partikulären superabsorbierenden Polymeren eine Reihe von Vorteilen auf:
i) So haben besagte Fasern oder Garne einen Dochteffekt Auf diese Weise können sie bei Kontakt mit einer Flüssigkeit sehr viel schneller diese Flüssigkeit aufnehmen und binden als dies partikuläre superabsorbierende Polymere können.
ii) überdies können die Flüssigkeitsströme lenken bzw. richten. Auf diese Weise kann z.B. eine Wundrandmazeration verhindert werden.
iii) Die Gefahr, dass superabsorbierende Materialien in der Wunde verbleiben, ist bei einem Gewebe aus superabsorbierenden Garnen sehr viel geringer als bei partikulären superabsorbierenden Polymeren. Auch dies trägt dazu bei, dass die superabsorbierenden Eigenschaften so sehr viel näher an den Wundgrund gebracht werden können.
iv) Da in vielen Fällen auf ein gesondertes Trägermaterial verzichtet werden kann, kann der Anteil an superabsorbierenden Materialien in dem Wundpflegeartikel erheblich erhöht werden.
v) Besagte Fasern oder Garne lassen sich zu einem Gefüge verarbeiten, ohne dass - wie es bei partikulären superabsorbierenden Polymeren erforderlich ist - ein Kleber oder ein Schweissverfahren verwendet werden muß. Dies hat sowohl in Bezug auf die Reinheit des Produkts als auch in Bezug auf die Pharmakologie und etwaige Allergenität erhebliche Vorteile.
vi) Im Gegensatz zu partikulären superabsorbierenden Polymere läßt sich die Dimensionierung besagter Fasern oder Garne sehr viel genauer steuern und kontrollieren, was einerseits zu Verhinderung von Stäuben führt, wie sie bei Verwendung von partikulären superabsorbierenden Polymere häufig entstehen, und was andererseits die Produktqualität (Homogenität und Reproduzierbarkeit) wesentlich erhöht.
Jegliche zwei- oder dreidimensionale Anordnung der Fasern oder Garne ist hier denkbar. So können die Fasern oder Garne gerichtet oder ungerichtet (Wirr-Warr), in mehreren Lagen oder sonstwie angeordnet sein.

Besagte Vliese können z.B. in Form eines Non-Woven-Wirbelvlieses vorliegen, wie es z.B. unter den Markennamen Alcantara, Dynamica, Jabana, Artinara, Bellseime oder Vivana vertrieben wird. Ebenso kann ein solches Vlies auch aus einer einfachen kardierten Mikrofaserwatte bestehen und/oder Elastan umfassen.

Ferner wird eine Ausfiihrungsform bevorzugt, in der die Mikrofasern zumindest abschnittsweise in einem Verbund mit anderen Fasern vorliegen.

Beispielsweise ist denkbar, dass die Mikrofasern als Monofil in einem Verbund mit saugfähigen Fäden ausgebildet sind, wobei die saugfähigen Fäden und die Monofile im wesentlichen gleich lang und in einem textilen Grundgewebe, Gewirke, Gestricke oder einer steifen Rueckenplatte durch Schweissen oder Verkleben verankert sind. Der Anteil der Monofile mag dabei 5 bis 50%, meist zwischen 10 und 40%, der Gesamtzahl von saugfähigen Fäden und Monofilen betragen. Zur Herstellung in Textiltechnik kommt dabei in erster Linie das Doppel-Plüsch-Verfahren in Betracht; die Monofile werden zusammen mit den saugfähigen Fäden als Pol in ein Grundgewebe eingearbeitet. Sie sollten in dem Grundgewebe durch Schweissen verankert sein, wofür z. B. in an sich bekannter Weise im Material des Grundgewebes schweissbare Fasern enthalten sein können. In dem geschweissten Gewebe sind die Monofile fest gehalten. Sie können sich nicht nach hinten heraus durchdrücken trotz der erheblichen Kraft, mit der sie sich infolge ihrer Steifigkeit in dem Gewebe abstützen. Durch die Verschweissung werden die Monofile außerdem an ihrem gesamten Umfang lückenlos eingefasst, so dass Biegemomente vollständig in das Gewebe übertragen werden und die Monofile nicht durch Verwinkelung gegenüber dem Grundgewebe abknicken können. Das Grundgewebe kann steif genug ausgeführt werden, um die Biegekräfte zu halten.

Insgesamt lassen sich durch gezielte Einstellung der Mischungsverhältnisse zwischen Mikrofasern und anderen Fasern, bzw. zwischen hydrophilen und hydrophoben Mikrofasern, die feuchtigkeitsleitenden bzw. feuchtigkeitsbindenden Eigenschaften des erfindungsgemäßen Wundpflegeartikels genau steuern.

Außerdem ist bevorzugt vorgesehen, dass die gasdichte Wundabdeckung eine fensterartige Behandlungsöffnung zur Entnahme des Wirkelements und/oder des Absorptionskörpers aufweist.

Falls der Wundpflegeartikel zur längeren Behandlung einer Wunde eingesetzt werden soll, ist es vorteilhaft, wenn das Wirkelement und/oder gegebenenfalls ein Absorptionskörper von unterhalb des Wundabdeckungselements entfernt werden kann. Dies kann je nach Beschaffenheit der Mikrofasern, des Absorptionsmaterials oder des Absorptionskörpers notwendig sein um zu gewährleisten, dass sich keine Bakterien im Wirkelement oder im Absorptionskörper ansiedeln.

Falls Absorptionsmaterial oder ein Absorptionskörper zur Unterstützung der Saugkraft des Unterdruck erzeugenden Mittels bzw. zur Aufrechterhaltung des Unterdrucks an der Wunde eingesetzt werden, kann es darüber hinaus erforderlich sein, das Wirkelement und/oder den Absorptionskörper auszutauschen, wenn die Absorptions- und/oder Retentionsfähigkeit des Absorptionsmaterials oder des Absorptionskörper überschritten ist.

In einer weiteren bevorzugten Ausführungsform weist der Absorptionskörper eine Hülle auf, deren Poren die Größe der superabsorbierenden Polymere nicht überschreitet. Die Porengröße richtet sich also nach der Größe der verwendeten Superabsorber-Teilchen, und kann dabei Werte von ≤ 50 bis ≤ 5000 µm annehmen, bevorzugt ≤ 100 bis ≤ 3000 µm.

Durch eine derartige Ausgestaltung wird erreicht, dass Wundsekrete, die durch die Hülle in den Absorptionsköper gelangt und von den superabsorbierenden Polymeren gebunden worden sind, innerhalb des Absorptionsköpers verbleiben, da die superabsorbierenden Polymere nicht durch die Hülle gelangen können. Somit würden in einer solchen Ausführungsform die abgesaugten Stoffe zum größten Teil aus Gasen bestehen, da Wundsekrete nur abgesaugt werden würden, falls die Absorptionskapazität des Absorptionskörpers erschöpft ist. Folglich eignet sich diese Ausführungsform besonders, falls der Aufbau des Wundpflegeartikels mit dem Einsatz von Unterdruck darauf abzielt, die Wirkungsweise des Absorptionskörper - Wundsekrete zu absorbieren - zu unterstützen und es nicht primär darum geht diese Sekrete abzusaugen. Wie oben beschrieben ist eine solche Vorgehensweise besonders bei Wunden, die zu Nekrosen neigen, angebracht, da die Unterdrucktherapie so sehr viel sanfter abläuft als mit herkömmlichen Wundpflegeartikeln zur Wundbehandlung unter Einsatz von Unterdruck.

Vorzugsweise ist die gasdichte Wundabdeckung überdies durchsichtig ausgestaltet, z.B. damit sich die Fortschritte im Wundheilungsprozess von außen beobachten lassen, damit darauf geachtet werden kann, dass es zu keiner Wundrandmazeration kommt und/oder man abschätzen kann, ob eventuell das Wirkelement oder der Absorptionskörper ausgewechselt werden müssen.

Bevorzugt wird zudem, dass das Wirkelement und/oder der Absorptionskörper zusätzlich Luftpolsterfolie umfasst.

Der Begriff "Luftpolsterfolie" bezeichnet ist eine elastische, und mindestens zweilagige Kunststofffolie aus Polyethylen, die zum Verpacken leicht zerbrechlicher Gegenstände benutzt wird.

Ein Wirkelement, das zusätzlich Luftpolsterfolie aufweist, ist bei besonders empfindlichen Wunden vorteilhaft, da die Folie wie eine dämpfende Polsterung gegen Einflüsse von Außen wirkt.

Weiterhin ist bevorzugt vorgesehen, dass die Wundauflage eine an anatomische Gegebenheiten angepasste Form aufweist. Hierzu kann die Wundauflage z.B. in Form einer Manschette ausgebildet sein, die über den einen Arm oder ein Bein oder ein Gelenk gestülpt werden kann, oder in Form eines an die Ferse, das Ellenbogengelenk oder dergleichen angepaßten Verbandes.

In einer möglichen Ausführungsform werden Gase und Flüssigkeit aus der Wunde abgesaugt. In einer derartigen Ausgestaltung ist bevorzugt vorgesehen, dass an der Einrichtung, über die in der Wunde befindliche Stoffe abgesaugt werden können, zwei Schläuche angeschlossen sind, wobei einer zur Erzeugung des Unterdrucks und einer zum Absaugen von Gase und Flüssigkeit dient.

In einer alternativen Ausführungsform wird ein erfindungsgemäßer Wundpflegeartikels in einer Vakuumtherapie zur Behandlung von chronischen Wunden verwendet.

Der beschriebene Wundpflegeartikel eignet sich besonders zur Vakuumtherapie zur Behandlung von chronischen Wunden da er im Stande ist, einen örtlich begrenzten negativen Druck in einer Wunde zu erzeugen. Dadurch wird der Heilungsprozess in chronischen Wunden beschleunigt, da ein eventuell bestehendes Wundödem verkleinert wird, die Durchblutung in der Wunde gefördert wird und die Wunde durch Befreiung von Wundsekreten gesäubert wird. In weiterer Folge bildet sich Granulationsgewebe, und eine feuchte Wundbehandlung ohne den Stau von Wundexsudat ist gegeben.

### ZEICHNUNGEN

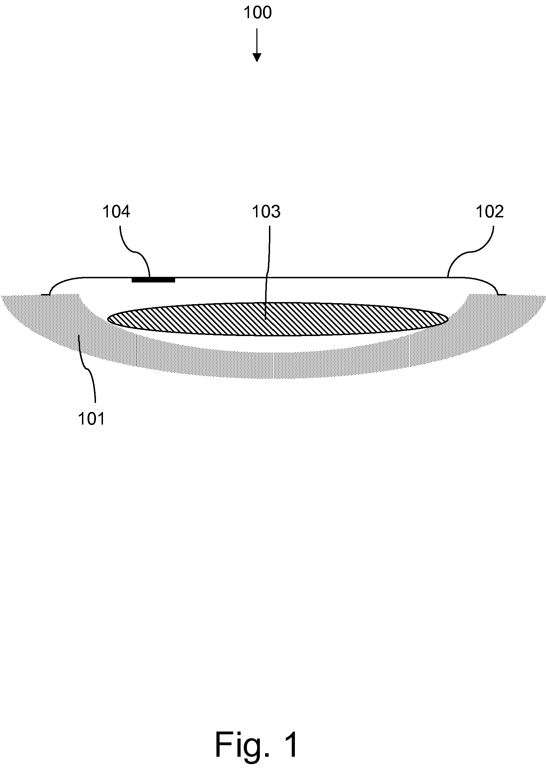

Fig. 1 zeigt einen nicht zur Erfindung gehörenden Wundpflegeartikel 100 vor der Erzeugung eines Unterdrucks. In diesem Beispiel umfasst der Wundpflegeartikel 100 eine Wundabdeckung 102, die um eine Wunde 101 eines Patienten herum angeordnet ist und diese Wunde gasdicht verschließt, ein Wirkelement 103, das unterhalb der Wundabdeckung 102 angeordnet ist und Mikrofasern aufweist, sowie eine Einrichtung 104, über die unter der Wundabdeckung befindliche Stoffe abgesaugt werden können.

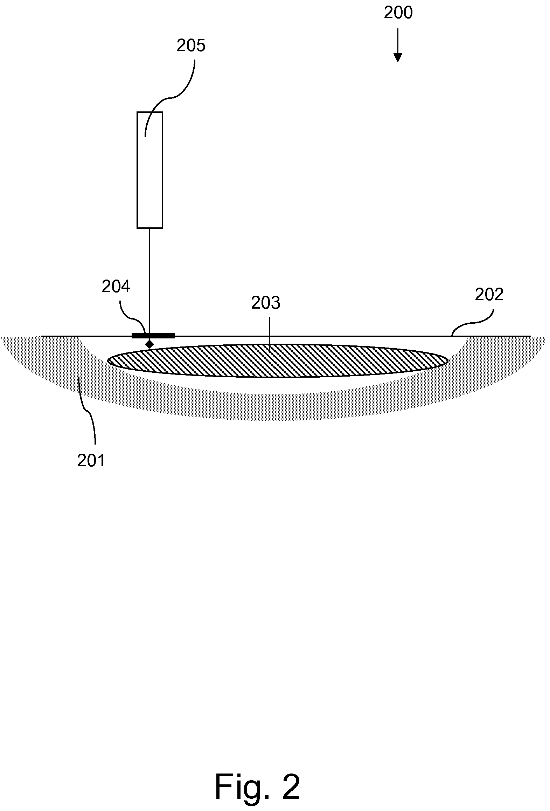

Fig. 2 zeigt einen nicht zur Erfindung gehörenden Wundpflegeartikel 200 aus Fig. 1 nach der Erzeugung eines Unterdrucks. Neben der Wundabdeckung 202, die um eine Wunde 201 eines Patienten herum angeordnet ist und diese Wunde gasdicht verschließt, dem Wirkelement 203, das unterhalb der Wundabdeckung angeordnet ist und Mikrofasern aufweist, sowie der Einrichtung 204, über die unter der Wundabdeckung 202 befindliche Stoffe abgesaugt werden können, umfasst dieser Wundpflegeartikel 200 auch ein Unterdruck erzeugendes Mittel 205, hier eine Injektionsspritze, mit der an der Einrichtung 204 die Luft unterhalb des Wundabdeckung abgesaugt wurde.

Durch den entstandenen Unterdruck hat sich die Wundabdeckung 202, zusammengezogen, das Wirkelement 203 ist jedoch nicht steif geworden, somit ist der Tragekomfort des Wundpflegeartikels 200 weiterhin gewährleistet.

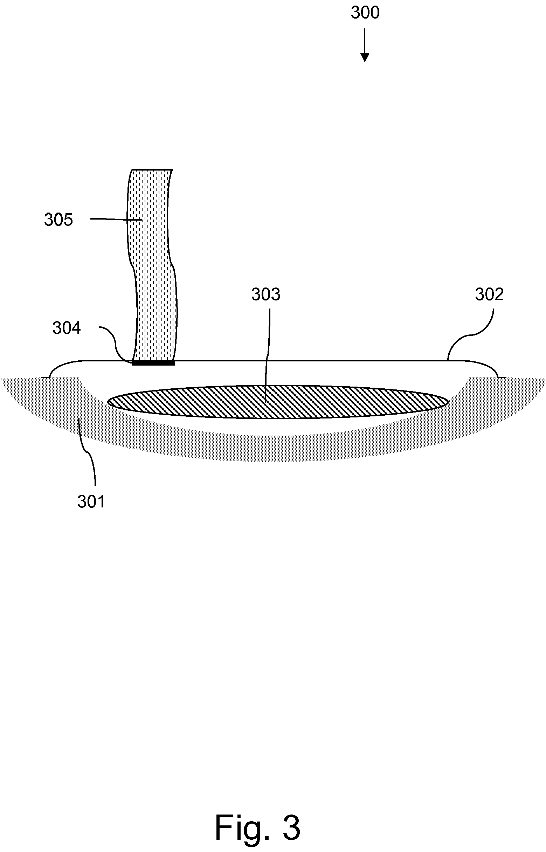

Fig. 3 zeigt einen nicht zur Erfindung gehörenden Wundpflegeartikel 300 umfassend einen Verbindungsschlauch 305 für eine Vakuumpumpe (nicht gezeigt) vor der Erzeugung eines Unterdrucks. In diesem Beispiel umfasst der Wundpflegeartikel 300 eine Wundabdeckung 302, die um eine Wunde 301 eines Patienten herum angeordnet ist und diese Wunde gasdicht verschließt, ein Wirkelement 303, das unterhalb der Wundabdeckung 302 angeordnet ist und Mikrofasern aufweist, und eine Einrichtung 304, an die der Verbindungsschlauch 305 für eine Vakuumpumpe angeschlossen ist.
Durch anschließen der nicht gezeigten Vakuumpumpe und anlegen eines Unterdrucks können in diesem Beispiel die unter der Wundabdeckung 302 befindliche Stoffe abgesaugt werden.

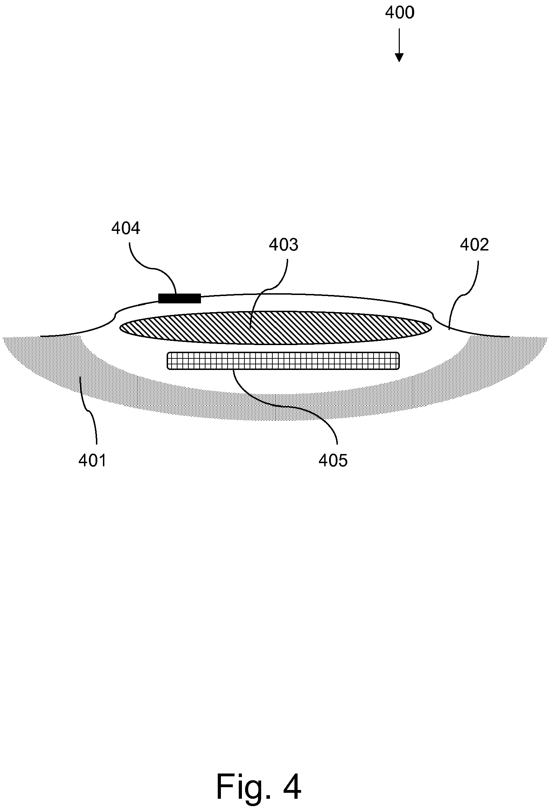

Fig. 4 zeigt einen erfindungsgemäßen Wundpflegeartikel 400 umfassend einen Absorptionskörper 405 aufweisend superabsorbierende Polymere nach der Erzeugung eines Unterdrucks.
Der erfindungsgemäßen Wundpflegeartikel 400 umfasst in diesem Bespiel eine Wundabdeckung 402, die um eine Wunde 401 eines Patienten herum angeordnet ist und diese Wunde gasdicht verschließt, ein Wirkelement 403, das unterhalb der Wundabdeckung 402 angeordnet ist und Mikrofasern aufweist, eine Einrichtung 404, über die unter der Wundabdeckung befindliche Stoffe abgesaugt wurden, und ein Absorptionskörper 405, der hier superabsorbierende Polymere aufweist und zwischen Wunde und Wirkelement 403 angeordnet ist.

In Fig. 4 ist über die Einrichtung 404 bereits Gas von unterhalb der Wundabdeckung 402 abgesaugt worden, so dass unterhalb der Wundabdeckung 402 nun ein Unterdruck herrscht. Dieser bewirkt, dass Wundsekrete vermehrt von dem Absorptionskörper 405, der superabsorbierende Polymere aufweist, absorbiert werden. Dadurch vergrößert der Absorptionskörper 405 sein Volumen und übt sowohl auf das Wirkelement 403 als auch auf die Wunde einen Druck aus. Der Druck auf die Wunde verhindert Wundrandmazeration. Das Wirkelement 403 versteift sich trotz Unterdruck und Druck durch den aufquellenden Absorptionskörper 405 nicht. Daher gibt es die Volumenänderung des Absorptionskörpers 405 an die Wundabdeckung 402 weiter, die sich folglich ausdehnt.

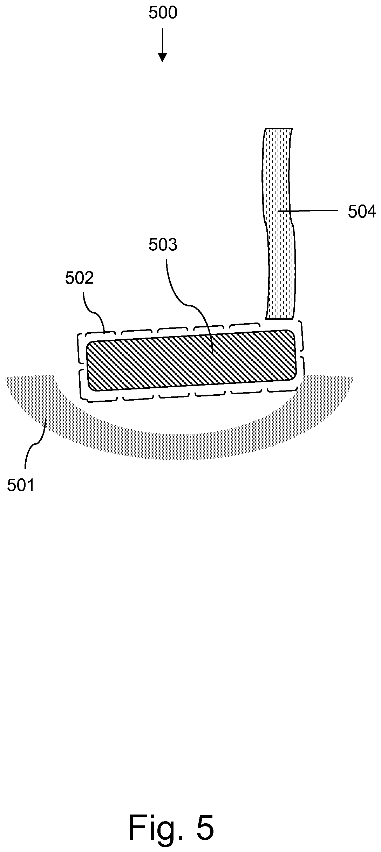

Fig. 5 zeigt einen nicht zur Erfindung gehörenden Wundpflegeartikel 500 der "wurstförmig" ausgestaltet ist - also eine Tamponade darstellt - und ein dreidimensionales Wunddistanzgitter 502 umfasst. Der Wundpflegeartikel 500 umfasst in diesem Bespiel ein als Tamponade ausgestaltetes Wirkelement 503 aufweisend Mikrofasern, das von einem dreidimensionales Wunddistanzgitter 502 umhüllt wird, sowie Verbindungsschlauch 504 für eine Vakuumpumpe (nicht gezeigt), über den die in der Wunde 501 befindliche Stoffe abgesaugt werden.

Diese Ausführungsform des Wundpflegeartikels 500 ist aufgrund ihrer großen Weichheit, die zur Schmerzreduktion beiträgt, und dem Umstand, dass das dreidimensionales Wunddistanzgitter 502 ein Verkleben mit dem Wundrand verhindert besonders für fistelartige Wunden und Wundtaschen geeignet.

## Patentansprüche

1. Wundpflegeartikel (100) zur Wundbehandlung unter Einsatz von Unterdruck aufweisend:
- ein Wirkelement aufweisend Mikrofasern (103), deren Einzelfaden feiner als 1 dtex sind, und
- eine Einrichtung (104), über die in der Wunde (101) befindliche Stoffe abgesaugt werden können,
wobei das Wirkelement aufweisend Mikrofasern auch ein Wundsekrete absorbierendes Absorptionsmaterial umfasst und der Wundpflegeartikel einen Wundsekrete absorbierenden Absorptionskörper (405) aufweist,
wobei das Wundsekrete absorbierende Absorptionsmaterial hydroaktive Polymere aufweist, und
wobei es sich bei den hydroaktiven Polymeren um superabsorbierende Polymere handelt.

2. Wundpflegeartikel gemäß Anspruch 1, ferner umfassend eine Wundabdeckung (102), mit der eine Wunde eines Patienten gasdicht verschließbar ist, wobei
- das Wirkelement (103) unterhalb der Wundabdeckung (102) anordenbar ist,
- die Einrichtung (104) in der Wundabdeckung (102) umfasst ist und so unter der Wundabdeckung (102) befindliche Stoffe abgesaugt werden können.

3. Wundpflegeartikel gemäß einem der Ansprüche 1-2, ferner umfassend wenigstens ein Unterdruck erzeugendes Mittel (205).

4. Wundpflegeartikel (100) gemäß einem der vorherigen Ansprüche, **dadurch gekennzeichnet,**
**dass** das Wirkelement aufweisend Mikrofasern mindestens auf einer Seite mindestens abschnittsweise in Kontakt mit einem Wunddistanzgitter (502) steht.

5. Wundpflegeartikel (100) gemäß einem der vorherigen Ansprüche, **dadurch gekennzeichnet, dass** das Wunddistanzgitter das Wirkelement aufweisend Mikrofasern in Form einer Hülle umgibt oder einen Teil einer solchen Hülle darstellt.

6. Wundpflegeartikel (100) gemäß einem der vorherigen Ansprüche, **dadurch gekennzeichnet, dass** das Wunddistanzgitter flächig zwischen Wunde und dem Wirkelement aufweisend Mikrofasern angeordnet ist.

## Claims

1. A wound care article (100) for the treatment of wounds making use of a vacuum and having:
• an active element containing microfibers (103) whose individual filaments are finer than 1 dtex, and
• a device (104) via which substances present in the wound (101) can be evacuated,
whereby the active element containing microfibers also comprises an absorbent material that absorbs wound secretions, and the wound care article has an absorption body (405) that absorbs wound secretions,
whereby the absorbent material that absorbs wound secretions contains hydroactive polymers, and
whereby the hydroactive polymers are superabsorbent polymers.

2. The wound care article according to claim 1, also comprising a wound covering (102) with which a wound of a patient can be sealed gastight,
whereby
• the active element (103) can be arranged below the wound covering (102),
• the device (104) is incorporated into the wound covering (102) so that substances present below the wound covering (102) can be evacuated.

3. The wound care article according to one of claims 1 or 2, also comprising at least one means (205) that generates a vacuum.

4. The wound care article (100) according to one of the preceding claims, **characterized in that**
the active element containing microfibers is in contact on at least one side, at least in certain sections, with a wound spacer lattice (502).

5. The wound care article (100) according to one of the preceding claims, **characterized in that**, in the manner of a sheath, the wound spacer lattice surrounds the active element containing microfibers or else it constitutes a part of such a sheath.

6. The wound care article (100) according to one of the preceding claims, **characterized in that** the wound spacer lattice is arranged to lie flat between the wound and the active element containing microfibers.

## Revendications

1. Article pour le traitement de plaies (100) destiné à traiter des plaies par l'utilisation d'une pression négative, comportant :
- un élément actif comportant des microfibres (103), dont les fils individuels ont une finesse inférieure à 1 dtex, et
- un dispositif (104) permettant d'aspirer des substances qui se trouvent dans la plaie (101),
l'élément actif comportant les microfibres comprenant également une matière absorbante qui absorbe des sécrétions de plaie et l'article pour le traitement de plaies comportant un corps absorbant (405) qui absorbe des sécrétions de plaie,
la matière absorbante qui absorbe des sécrétions de plaie comportant des polymères hydroactifs et
les polymères hydroactifs étant des polymères superabsorbants.

2. Article pour le traitement de plaies selon la revendication 1, comprenant en outre un pansement (102) qui permet de fermer une plaie d'un patient de manière étanche aux gaz,
- l'élément actif (103) pouvant être agencé en dessous du pansement (102),
- le dispositif (104) étant compris dans le pansement (102) et des substances situées en dessous du pansement (102) peuvent ainsi être absorbées.

3. Article pour le traitement de plaies selon l'une des revendications 1-2, comprenant en outre au moins un moyen (205) qui génère une pression négative.

4. Article pour le traitement de plaies (100) selon l'une des revendications précédentes, **caractérisé en ce que** l'élément actif comportant les microfibres est en contact, sur au moins un côté, au moins en partie, avec une grille d'espacement de plaie (502).

5. Article pour le traitement de plaies (100) selon l'une des revendications précédentes, **caractérisé en ce que** la grille d'espacement de plaie entoure sous forme d'enveloppe l'élément actif comportant les microfibres ou représente une partie d'une telle enveloppe.

6. Article pour le traitement de plaies (100) selon l'une des revendications précédentes, **caractérisé en ce que** la grille d'espacement de plaie est agencée à plat entre la plaie et l'élément actif comportant les microfibres.
